# EUROPEAN PATENT APPLICATION

(11) **EP 0 839 915 A1**
(43) Date of publication of application: **06.05.1998**
(21) Application number: 96203051.6
(22) Date of filing: 01.11.1996
(51) Int. Cl.: C12N 15/62, C07K 16/00, C07K 16/46, C07K 1/22, A61K 39/385, A61K 39/395

(54) **Secretory immunoglobulin A as a mucosal vaccine delivery system**

(71) Applicant: INSTITUT SUISSE DE RECHERCHES EXPERIMENTALES SUR LE CANCER ISREC, 1066 Epalinges s/Lausanne (CH)
(72) Inventor: Kraehenbuhl, Jean-Pierre, 1812 Rivaz (CH); Corthésy, Blaise, 1066 Epalinges (CH); Kaufmann, Muriel, 1073 Savigny (CH); Peitsch, Manuel, 1866 La Forclaz (CH)
(74) Representative: Arnold, Winfried

(57) **Abstract**

A secretory IgA antibody (sIgA) comprising a polymeric IgA antibody bound to a secretory component (SC) which SC comprises a (poly)peptide insert corresponding to a protective epitope of an antigen; a vaccine comprising such sIgA antibody; a DNA construct comprising a DNA sequence coding for a SC and a DNA insert coding for the protective epitope of an antigen; a method for producing said sIgA antibody; and a method for active immunization of a mammal which comprises administering to said mammal an effective amount of a vaccine comprising said sIgA.

## Description

### Field of the Invention

The present invention relates to a novel secretory immunoglobulin A in which the secretory component is of chimeric nature, novel and comprises an immunogenic epitope which is foreign to the parent secretory component, said novel chimeric secretory component, methods of producing said secretory immunoglobulin A and said secretory component, the use of these compounds in a novel vaccine delivery system for the vaccination of a mammal, and pharmaceutical compositions comprising said secretory immunoglobulin A.

### Background of the Invention

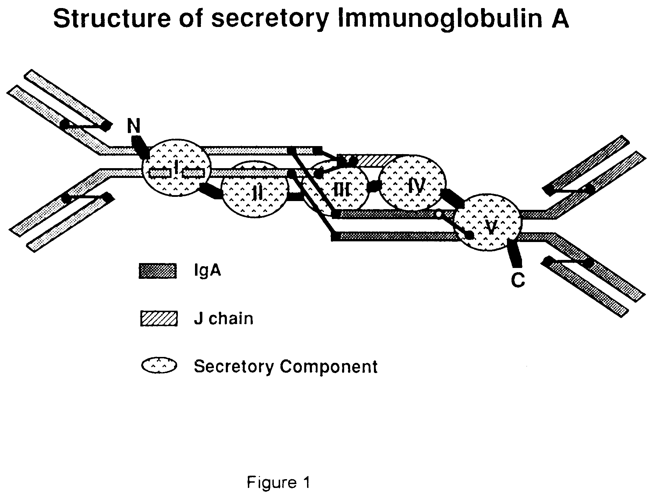

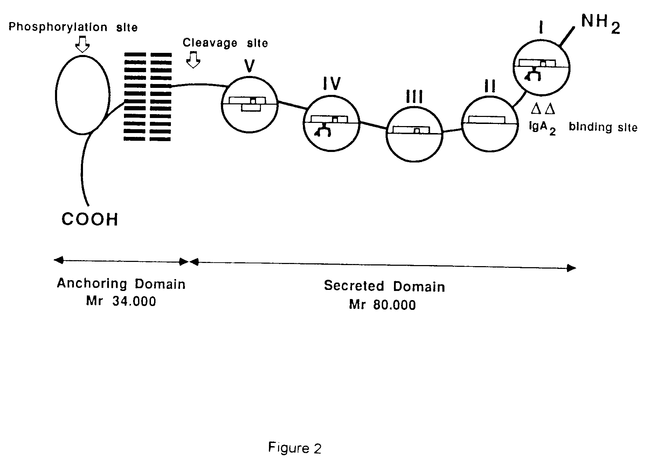

The protection of the mucosal surfaces of the digestive, respiratory and urogenital tracts is in part mediated by secretory immunoglobulin A (sIgA; Fig. 1). This antibody consists of an IgA dimer associated with J chain, which is acquired during the process of polymerization in plasma cells just before secretion, and secretory component (SC). SC is derived from the polymeric immunoglobulin receptor (PIR; Fig. 2), which binds and transports polymeric immunoglobulins across mucosal and glandular epithelia. Proteolytic cleavage of the extracellular portion of the receptor releases SC together with the bound IgA into mucosal secretions (Neutra et al., 1994). SC belongs to the immunoglobulin superfamily of proteins, and consists of a series of five Ig-like domains corresponding to the portion of the poly-Ig receptor facing the outside (Mostov et al., 1984; Banting et al., 1989; Schaerer et al., 1990; Krajci et al., 1991).

In addition to the protection of mucosal surfaces by crosslinking pathogens and promoting their clearance by peristalsis or mucociliary movement, sIgA in the intestinal lumen also selectively adheres to M cells (these cells transport antigens across the epithelium overlying organized lymphoid mucosal tissue). This was first observed in suckling rabbits as a local accumulation of milk sIgA on M cells of Peyer's patches (Roy and Varvayanis, 1987). Subsequently, monoclonal mouse IgA, polyclonal rat IgA, and polyclonal IgG antibodies, radiolabeled or coupled to colloidal gold, were found to bind specifically to rabbit or mouse M cells and to compete with each other for binding sites (Weltzin et al., 1989). Selective sIgA binding and transport by M cells may play a role in the regulation of the mucosal immune response, but that role has not yet been defined. Thus, while sIgA would generally prevent the contact of antigens with mucosal surfaces, it could also promote re-uptake of small amounts of antigen by M cells.

Up to now it was not known whether oral application of sIgA carrying a foreign epitope ("antigenized" sIgA) could elicit an immunogenic response against the inserted epitope and whether recombinant chimeric SC-IgA complexes can serve as mucosal vaccine delivery systems.

### Object of the Invention

It is an object of the invention to take advantage of the fact that orally administered sIgAs are efficiently transported by M cells in Peyer's patches of the small intestine and in other mucosa-associated lymphoid tissue (MALT) of the large intestine, the oral and nasal cavities, and the airways and the possibility that sIgA itself could serve as a vaccine delivery vector to target foreign epitopes into MALT. However, this would require that the foreign epitope be inserted without affecting the molecular folding of SC or the assembly and the function of sIgA. Also, the site of insertion must be surface-exposed, and the inserted epitope must be immunogenic.

The object of this invention is to solve these problems and to generate sIgA comprising a foreign epitope in the secretory component which foreign epitope does not affect the molecular folding of SC or the assembly and the function of sIgA, which foreign epitope is inserted in a surface-exposed site of the SC, and which inserted foreign epitope is immunogenic.

### Summary of the Invention

Secretory IgA was already known to bind to M cells and to be transported into the underlying organized mucosal lymphoid tissue (Weltzin et al., 1989). To act as a vaccine carrier able to deliver foreign epitopes, sIgA was processed and presented to the cells of the immune system. Surprisingly it was found to trigger a systemic and a mucosal response. To show that orally administered sIgA from rabbit whey is taken up and processed in mouse Peyer's patches, lymphoblasts in these latter are recovered from immunized animals, fused with myeloma partners to generate hybridoma cells (Weltzin et al., 1989) secreting immunoglobulins specific for rabbit SC and sIgA. In the particular example, 10 hybridoma clones out of 60 react with SC by ELISA, out of which four recognize free and bound SC in rabbit whey on Western blots. One clone produces polymeric IgM, two clones secrete IgG1, and one clone secretes IgA_{d}. IgA_{d} from clone 36.4 recognizes both free and IgA-bound SC blotted on nitrocellulose membrane and immunoprecipitate free SC when immobilized on Sepharose beads. This indicates that mucosally administered sIgA is able to elicit both a mucosal and systemic immune response against SC.

The present invention demonstrates that oral immunization with "antigenized" sIgA elicits both a systemic and mucosal response against the inserted epitope and that recombinant chimeric SC-IgA complexes can serve as mucosal vaccine delivery systems.

### Detailed Description of the Invention

The invention concerns a secretory IgA antibody comprising a polymeric IgA antibody bound to a secretory component which comprises a (poly)peptide insert corresponding to a protective epitope of an antigen.

The invention concerns further a vaccine comprising an effective amount of a secretory IgA comprising a polymeric IgA antibody bound to a secretory component which comprises a (poly)peptide insert corresponding to a protective epitope of an antigen.

The invention concerns further a chimeric secretory component which comprises as a (poly)peptide insert a protective epitope of an antigen.

The invention concerns further a secretory component as described hereinbefore and hereinafter wherein the (poly)peptide insert is a protective epitope of a pathogenic microorganism.

The invention concerns further a secretory component as described hereinbefore and hereinafter, wherein the (poly)peptide insert is an amino acid sequence from a bacterial, viral, parasitic or mammalian antigen which is recognized by an immune effector, i.e. antibody, antigen presenting cell or lymphocyte.

The invention concerns further a DNA construct comprising a DNA sequence coding for a secretory component and a DNA insert coding for the protective epitope of an antigen.

The invention concerns further a method for producing a secretory IgA antibody comprising a polymeric IgA antibody bound to a secretory component, which comprises a peptide insert corresponding to a protective epitope of an antigen, wherein said secretory component is combined with said polymeric IgA antibody.

The invention concerns further a method for active immunization of a mammal which comprises administering to said mammal an effective amount of a vaccine comprising a secretory IgA comprising a polymeric IgA antibody bound to a secretory component which comprises a peptide insert corresponding to a protective epitope of an antigen.

### Brief Description of the Figures

Sequence identification numbers (SEQ ID NO) are abbreviated in the Figures as IdNo.
Fig. 1. Schematic representation of secretory immunoglobulin A. The protein is made of two immunoglobulin A monomers covalently linked through the J chain, together with secretory component wrapped around the surface of the molecule.
Fig. 2. Schematic representation of the polymeric immunoglobulin receptor (PIR). - NH₂: The amino terminus of PIR. -COOH: The carboxyl terminus of PIR. Spheres numbered I to V: the five IgG-like extracellular domains of the receptor, with some important disulfide bridges and glycosylation sites drawn. Secretory component (SC) comprises the N-terminus amino acid down to the cleavage site.
Fig. 3. Scheme decribing the sites of epitope replacement/insertion within secretory component (SC). A: Rabbit SC. B: Mouse SC. The sites are expressed as amino acid numbers, with respect to the initiation methionine given number 1. Empty box: Signal sequence. Filled box: Sites of insertion/replacement.
Fig. 4. Scheme (not drawn on scale) describing the construction of plasmid pSC-11(A/M).
Fig. 5. Scheme (not drawn on scale) describing the construction of plasmids pSC(IpaB), pHGS1-SC, pHGS1-SC(IpaB), pHGS1-SC6xHis, pHGS1-SC(IpaB)6xHis, pLKneo-PIR(IpaB).
Fig. 6. Purification of SC(IpaB) by Ni²⁺-chelate affinity chromatography. L: Load; F: Flow through; W: Wash; E40: Elution with 40 mM imidazole; E80: Elution with 80 mM imidazole. Panel A: The purified protein as appearing after silver staining. Panel B: The purified protein as appearing after immunodetection (Western blot).
Fig. 7. Association of PIR(wt) and PIR(IpaB) with IgA_{d}.
   Panel A: Cartoon representation of dot blot reassociation assay (DORA). Further description of the procedure is available in Rindisbacher et al. (1995).
   Panel B: Both the wild-type and chimeric SC protein produced in rVV-infected CV-1 cells bind to IgA_{d} with comparable affinity. No interaction occurs when IgAₘ is used as a partner in the reconstitution reaction. Anti-α chain: monoclonal antibodies to the heavy chain of IgA. Anti-SC: pAb 982 to the rabbit secretory component. Detection was performed using secondary antibodies labeled with HRP, and enhanced chemioluminescence.

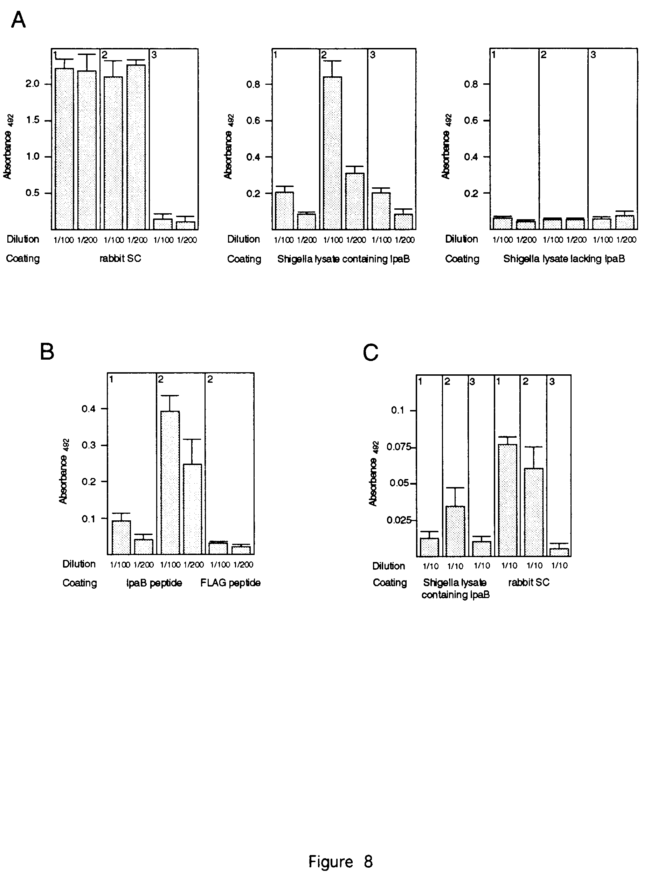

Fig. 8. Oral immunogenicity in BALB/c mice of antigenized sIgA.
   Panel A: Binding specificity of the mouse sera to the indicated antigen (noted below each panel) measured by ELISA. Groups of mice are: 1, mice immunized with IgA_{d}-SC(wt); 2, mice immunized with IgA_{d}-SC(IpaB); 3, mice given PBS (negative control). Results are mean ± standard deviation of two separate triplicate experiments involving groups of three mice, and are expressed as absorbance values at 492 nm.
   Panel B: Binding specificity of the mouse sera to the specific IpaB peptide and control FLAG peptide, measured by ELISA. Groups of mice are: 1, mice immunized with IgA_{d}-SC(wt); 2, mice immunized with IgA_{d}-SC(IpaB); 3, mice given PBS (negative control). Results are mean ± standard deviation of two separate triplicate experiments involving groups of three mice, and are expressed as absorbance values at 492 nm.
   Panel C: Binding specificity of saliva samples recovered from mice in groups 1, 2 and 3.

### Short description of sequences used according to Sequence Listing

SEQ ID NO 1: Oligonucleotide for PCR, noncoding strand, insertion of an AccI site at position 418, hybridizing sequence 429-394 in pSC-11, used to generate pSC-11(A/M).
SEQ ID NO 2: Oligonucleotide for PCR, coding strand, hybridizing sequence 314-337 in pSC-11, used to generate pSC-11(A/M).
SEQ ID NO 3: Oligonucleotide for PCR, coding strand, insertion of a MroI site at position 453, hybridizing sequence: 448-480 in pSC-11, used to generate pSC-11(A/M).
SEQ ID NO 4: Oligonucleotide for PCR, noncoding strand, hybridizing sequence: 905-881 in pSC-11, used to generate pSC-11(A/M).
SEQ ID NO 5: Oligonucleotide coding for the Shigella invasin B (IpaB) epitope KDRTLIEQK (coding strand) with a 5' AccI half site for insertion into pSC-11(A/M) and generation of pSC(IpaB).
SEQ ID NO 6: Oligonucleotide coding for the Shigella invasin B (IpaB) epitope KDRTLIEQK (noncoding strand) with a 3' MroI half site for insertion into pSC-11(A/M) and generation of pSC(IpaB).
SEQ ID NO 7: Oligonucleotide coding for a six histidine tag (coding strand) for insertion into pHGS1-SC and generation of pHGS1-SC6xHis.
SEQ ID No 8: Oligonucleotide coding for a six histidine tag (coding strand) for insertion into pHGS1-SC and generation of pHGS1-SC6xHis.
SEQ ID NO 9: Upstream primer for PCR analysis.
SEQ ID NO 10: Downstream primer for PCR analysis.
SEQ ID NO 11: Amino acid sequence of Shigella invasin B epitope.

The terms used in this description and the claims are further defined as follows:

A secretory IgA antibody (sIgA) according to the invention is a novel antibody comprising a polymeric IgA antibody bound to a secretory component which comprises a (poly)peptide insert corresponding to a protective epitope of an antigen, and which is able to trigger an immune response to the protective epitope in a mammal to which an effective amount has been administered. It is able to be transported across mucosal and glandular epithelia.

Polymeric IgA antibodies are known in the art or can be prepared according to known procedures. Such antibodies are usually dimeric or polymeric. Representative examples are in particular monoclonal antibodies from hybridoma cell lines, e.g. from ZAC3 hybridoma (anti-*Shigella flexneri* lipopolysaccharide), 2D6 hybridoma (anti-*Vibrio cholerae* lipopolysaccharide), IgA71 hybridoma (anti-*Helicobacter pylori* urease), IgAC5 (anti-*Shigella flexneri* lipopolysaccharide), Sal4 hybridoma (anti-*Salmonella typhimurium*), IgA380 hybridoma (anti-Sendai virus hemagglutinin-neuraminidase), IgA 59 hybridoma (anti-influenza virus hemagglutinin), HNK-20 hybridoma (anti-respiratory syncytial virus).

A secretory component (SC) comprised by a secretory IgA antibody according to the invention, comprising a peptide insert corresponding to a protective epitope of an antigen, also called a chimeric SC, and which is able to trigger an immune response to the protective epitope is a novel chimeric SC. It is obtainable by recombinant DNA techniques in that a DNA sequence coding for an epitope which is foreign to the parent SC is inserted at a selected position of the parent SC so that the finally expressed foreign chimeric epitope in the secretory component does not affect the molecular folding of the novel SC, the assembly with polymeric IgA and the function of the novel sIgA as a vaccine delivery system.

The parent SC can be any SC, in particular one already known in the art, and which may be readily available, such as for example the rabbit, the mouse, the rat and the human SC.

In the present examples, we selected rabbit SC for epitope insertion because 1). the cDNA for the SC portion of pIgR was available (Schaerer et al., 1990), 2). the SC was shown not to perturb the binding of the sIgA to the antigen (Lüllau et al., 1996), 3). the SC and the IgA_{d} can combine to form a sIgA *in vitro* (Rindisbacher et al., 1995), and 4). a battery of monoclonal and polyclonal antibodies to the SC is available to map the possible effects of epitope substitution on protein structure (Table 1: see below). Our results first show a so far not identified function of domain I in the process of SC secretion, which nonetheless did not preclude either overexpression, or IgA binding of chimeric SC.

Foreign protective epitopes likewise can be any one retaining its protective, i. e. antigenic and immunogenic function after insertion. They are for example selected from antigenic surface proteins of pathogenic fungi, bacteria, virus, parasites, or toxins, e.g. from intestinal, respiratory or genital bacterial pathogens, such as *Shigella*, *Yersinia*, *Vibrio cholerae*, *Salmonella*, *Streptococcus pneumoniae*, *Neisseriae*, *or Chlamydia,* from intestinal, respiratory or genital viruses, such as rotavirus, coronavirus, influenza, virus, respiratory syncytial virus, herpes viruses, human papilloma virus, or human immunodeficiency virus, from intestinal, respiratory, genital toxins, such as pertussis toxin, diphteria toxin, cholera toxin, botulinal toxin, heat labile or stable toxins, or from intestinal, respiratory or genital parasites, such as shistosomia or giardia. They have for example a length of about 9 up to 21 or more, preferably about 8 or 9 amino acids, and are preferentially hydrophilic for insertion on the surface of the carrier protein. Such epitopes are for example the *Shigella* invasin epitope of IpaB of the sequence KDRTLIEQK, SEQ ID NO 11, or the immunogenic epitopes of the third variable (V3) loop of HIV-1 envelope protein gp 120 in the region of between amino acid Nos. 309 to 318 (Javaherian et al., 1989), an Epstein-Barr virus nuclear antigens in the region of between amino acid Nos. 64 to 83 (antigen 2; Schmidt et al., 1991), Nos. 339 to 347 (antigen 3; Burrows et al., 1990a), Nos. 416 to 424 (antigen 4; deCampos-Lima et al., 1994), or Nos. 290 to 299 (antigen 6; Burrows et al., 1990b), an influenza A virus hemagglutinin epitope in the region of between amino acid Nos. 110 to 120 (Zaghouani et al., 1992), an influenza virus nucleoprotein epitope in the region of between amino acid Nos. 147 to 161 (Taylor et al., 1987), a bacteriophage 1 cI repressor protein in the region of between amino acid Nos. 12 to 26 (Guillet et al., 1986), a diphteria toxin variant CRM₁₉₇ in the region of between amino acid Nos. 366 to 383 (Bixler et al., 1989), a tetanus toxin P30 epitope in the region of between amino acid Nos. 947 to 967 (Panina-Bordignon et al., 1989), a mouse mammary tumor virus gp52 epitope in the region of between amino acid Nos. 143 to 153 (Dion et al., 1990), and in particular immunogenic epitopes of *Helicobacter pylori*, such as the urease B subunit (Labigne et al., 1991) or fragments thereof, e. g. in the region of between amino acid Nos. 220 to 569, or Nos. 220 to 344, and the heat-shock protein GroES (Suerbaum et al., 1994) or fragments thereof, in the region of between amino acid Nos. 1 to 118. It is also possible to insert mimotopes. For instance, bacterial lipopolysaccharides epitopes can be converted into peptide mimotopes using monoclonal antibody screening of peptide phage libraries (Hoess et al., 1993). Foreign epitopes are preferably inserted in a position of the parent SC where they do not inadequately interfere with the natural folding of the SC, where they do not inhibit the binding to the polymeric, especially dimeric IgA in vitro, and where they still retain their antigenic and immunogenic activity. The protective epitope may completely or partially replace a SC domain or be fused to the carboxy or amino terminus of the SC.

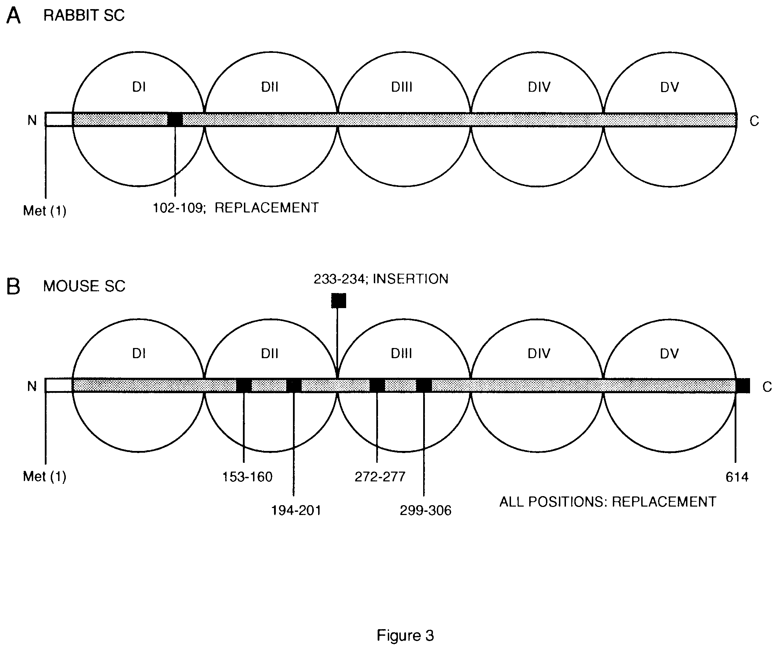

For example such position is a surface-exposed site of the loop connecting the E and F β strand of domain I of rabbit SC. In particular the eight amino acid sequence corresponding to position 102-109 in the protein (Fig. 3) can be replaced by the nine amino acid foreign epitope derived from *Shigella* IpaB, SEQ ID NO 11. Further prefered insertion sites are situated in domain II and III, in particular at the positions of between amino acid Nos. 153 to 160, Nos. 194 to 201, Nos. 233 to 234, Nos. 272 to 277, Nos. 299 to 306, and the carboxy terminus, respectively, in the mouse SC sequence (Fig. 3). The foreign epitope may be inserted in an operable position of the SC amino acid sequence, provided that the overall structure can tolerate such a replacement. It is of high probability that, due to the very conserved sequence between SC genes, a site mapped in one species will work in others.

The invention concerns further a vaccine comprising a secretory IgA comprising a polymeric IgA antibody bound to a secretory component which comprises a peptide insert corresponding to a protective epitope of an antigen. The vaccine of the present invention is in the form of a pharmaceutical composition of conventional nature. A pharmaceutical preparation according to the present invention for the prevention or treatment of an infection of mucosal lymphoid tissue in a mammal, including humans, comprises an effective amount of a secretory IgA antibody (sIgA) according to the invention, eventually purified according to conventional methods, and a conventional pharmaceutical carrier. The pharmaceutical preparation can be used nasally, genitally or rectally and in particular orally, in liquid form, or completely dried in the form of a powder, which can be pressed into tablets, filled into capsules, or applied as a dry powder in the form of a spray, e. g. nasal spray. Suppositories or ointment are useful for rectal or vaginal application. Conventional production methods are applied making use of conventional pharmaceutical carriers.

The method of protecting a mammal, including humans, against an infectious organism by active or passive immunization according to the present invention comprises administering of effective amounts of the pathogen-specific sIgA carrying the foreign chimeric epitope. The pharmaceutical vaccine is administered nasally, genitally, rectally or preferably orally for those pathogens that infect mucosal surfaces of the respiratory, urogenital or digestive tracts. Pharmaceutical preparations in acid stable capsules can be used in case of intestinal infections. Pharmaceutical preparations in form of nose drops or sprays are used when nasal administration is required, e.g. in the case of respiratory infections. Suppositories or ointment are applied in case of rectal or vaginal infections.

The pharmaceutical preparations have to be administered according to the judgement of the physician in effective amounts depending on the concentration of the sIgA and the route of administration so that a protective antigenic, immunogenic or curative effect is obtained. The amounts and method of administration are to be selected further depending upon the age and weight of the patient, the nature and severity of the infection as well as the general condition of the patient. In general it is sufficient to administer the sIgA in amounts of about 1 to 50 milligrams, e.g. orally or nasally, per patient either once or several, e.g. four times within one or two months.

The invention concerns further a secretory component (SC) of an IgA which comprises as a (poly)peptide insert a protective epitope of an antigen as defined hereinbefore, in particular wherein the peptide insert is an amino acid sequence from a bacterial, viral or parasitic antigen **or a toxin antigen** which is recognized by an antibody.

Such SCs are prepared according to conventional recombinant DNA techniques, whereby a DNA insert coding for the epitope is prepared, e. g. by chemical synthesis, inserted in proper reading frame into the DNA coding for the SC in a position defined hereinbefore to produce a chimeric DNA coding for the SC and the foreign epitope. The chimeric DNA is inserted into a proper expression vector which is then introduced into a proper mammalian cell where it is converted under suitable conditions into chimeric, glycosylated SC protein. The expressed chimeric SC comprising the foreign antigenic and immunogenic protective epitope(s) is recovered and purified according to conventional methods, e.g. by Ni²⁺-chelate affinity chromatography (Rindisbacher et al., 1995).

The invention concerns further a chimeric DNA construct comprising a DNA sequence coding for a secretory component of an IgA and a DNA insert coding for the protective epitope of an antigen. Such construct is in particular an expression vector able to express the chimeric SC in an eukaryote, in particular in a mammalian, including a human, cell. Representative constructs are coding sequences inserted into expression vectors including pCB6, pCB7, pcDNA3, pCI, pSI, recombinant vaccinia viruses, and recombinant baculoviruses.

The invention concerns further a method for producing a secretory IgA antibody comprising a polymeric IgA antibody bound to a chimeric secretory component, which comprises a peptide insert corresponding to a protective epitope of an antigen, wherein said secretory component is combined with said polymeric IgA antibody. The polymeric IgA and the chimeric SC are in particular such as defined hereinbefore. The combination of the two components, in particular of dimeric IgA and chimeric SC to give the sIgA is preferably carried out in vitro as described by Rindisbacher et al. (1995). Briefly, 1 microgram of purified recombinant SC in PBS buffer and 5 micrograms of purified dimeric IgA in PBS buffer are mixed together in 100 microliters (final volume) of PBS buffer, and allowed to reassociate at ambient temperature for 16 hours.

The invention concerns further a method for active immunization of a mammal which comprises administering to said mammal of an effective amount of a vaccine comprising a secretory IgA comprising a polymeric IgA antibody bound to a secretory component which comprises a (poly)peptide insert corresponding to a protective epitope of an antigen.

### Abbreviations

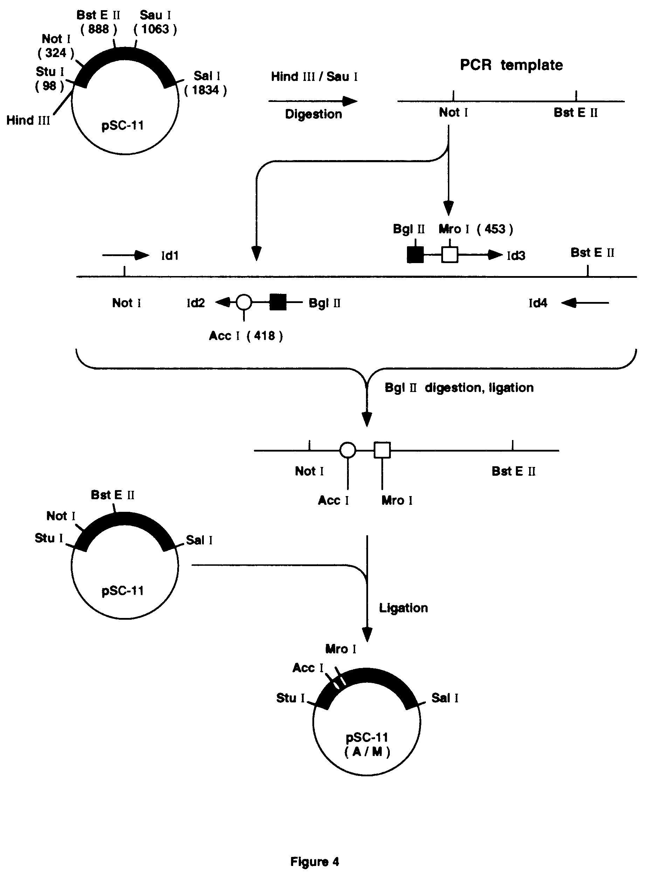

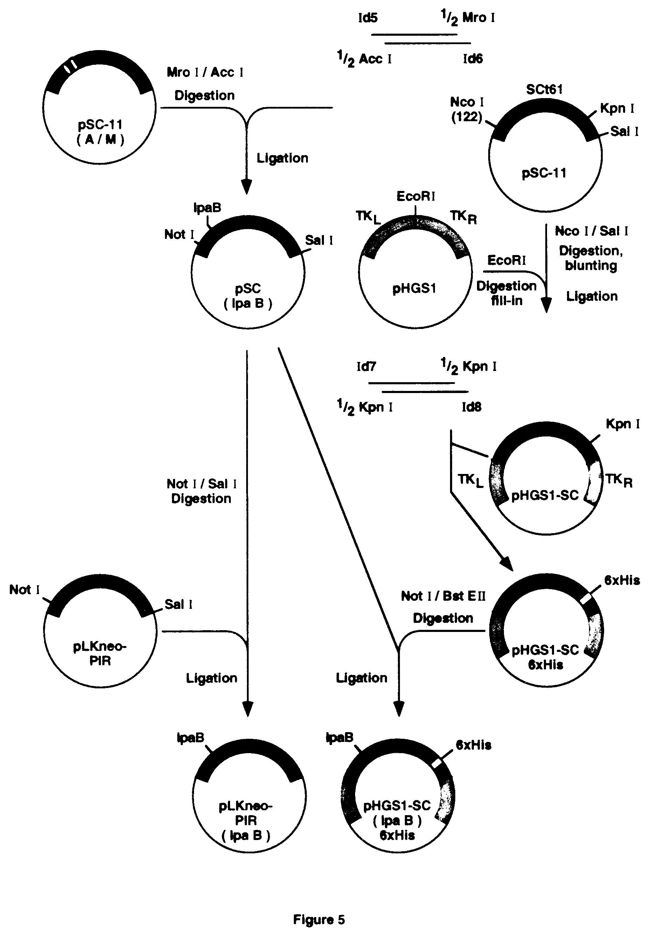

Throughout the entire description the following abbreviations are used:
cDNA complementary DNA
CV-1 African green monkey kidney cells (ATCC CCL70)
ELISA enzyme linked immunosorbent assay
FPLC fast protein liquid chromatography
HRP horse radish peroxidase
IgAₚ IgA polymer
IgA_{d} dimeric IgA
IgA 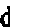 _{-SC(IpaB)} dimeric IgA with secretory component contaning IpaB
IgA 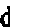 _{-SC(wt)} dimeric IgA with wild type secretory component
IgA monomeric IgA
IpaB Shigella invasin protein antigen B
M cells cells transporting antigens across epithelium overlaying organized lymphoid tissue
MALT mucosa-associated lymphoid tissue
p11K late promoter of the 11K envelope protein of vaccinia virus
pAb polyclonal antibody
PAGE polyacrylamide gel electrophoresis
PBS phosphate buffered saline, pH 7.2
PCR polymerase chain reaction
pHGS1-SC plasmid Fig. 5
pHGS1-SC6xHis plasmid Fig. 5
pIBI-SC(IpaB) plasmid Fig. 5
pSC-11(A/M) plasmid Fig. 4
pSC-11 plasmid Fig. 4, (Schaerer et al., 1990)
PIR polymeric immunoglobulin receptor
PIR(IpaB) PIR comprising the IpaB epitope
PIR(wt) PIR wild type
pLKneo-PIR(IpaB) plasmid Fig. 5
pLKneo-PIR plasmid Fig. 5
rVV recombinant vaccinia virus
SC secretory component
SC(IpaB) secretory component comprising the IpaB epitope
SC-IgA complex of secretory component and IgA
SDS sodium dodecylsulfate
sIgA secretory immunoglobulin A
TK_{L} thymidine kinase, left arm of vv TK gene
TK_{R} thymidine kinase, right arm of vv TK gene
Tris-HCl tris(hydroxymethyl)aminomethane with hydrogene chloride
vv vaccinia virus
vvHGS1-SC(IpaB)6xHis recombinant vaccinia virus used for the expression of SC(IpaB)6xHis
vvHGS1-SC6xHis recombinant vaccinia virus used for the expression of SC6xHis
wt wild type
ZAC 3 hybridoma cell line secreting IgA antibodies

### Monoclonal antibodies (mAb) and polyclonal antisera (pAb):

The monoclonal antibodies (mAb) and polyclonal antisera (pAb) used in the examples and their properties are listed in Table 1.

**Table 1**

| **Antiserum/antibody** | **Target** | **Detection** | **References** |
|---|---|---|---|
| goat pAb 982 | SC, PIR sIgA | ELISA, IP, Western | Solari et al. 1985 |
| rabbit pAb anti-t61 allele | SC, sIgA | Western | Hanly et al. 1987 |
| guinea pig pAb | SC domains II and III | Western | Schaerer et al. 1990 |
| mouse mAb 303 | SC domain SC, sIgA | IP, Western | Kuhn et al. 1983 |
| mouse mAb 166 | PIR cytoplasmic tail | IP, Western | Kuhn et al. 1983 |
| mouse mAb H10 | invasinB KDRTLIEQK epitope | IP, Western | Barzu et al. 1993 |
| Abbreviations: pAb: polyclonal antiserum; mAb: monoclonal antibody; IP: immunoprecipitation; ELISA: enzyme linked immunosorbent assay. | | | |

The following examples serve to further illustrate the invention without restricting it.

In the examples the following materials and general methods were used.

### Example 1: Construction of chimerized SC cDNA plasmid pSC(IpaB).

A rabbit PIR cDNA fragment (allele t61) is recovered from plasmid pSC-11 (Fig. 4, Schaerer et al., 1990) by HindIII/SauI digestion. The 1082 bp fragment serves as a template for two PCRs aiming at the insertion of two unique restriction sites for subsequent sequence replacement within domain I of the rabbit SC clone. PCR conditions are as follows: 94°C for one minute, 58°C for 1 minute, and 72°C for 2 minutes, repeated for 30 cycles using a thermocycler from Cetus Corporation. In the first reaction, an AccI site is created at position 418 using oligonucleotides
SEQ ID NO 1: (hybridizing sequence: 429-394) and
SEQ ID NO 2: (hybridizing sequence: 314-337).

In order to facilitate subsequent ligation of PCR products, the 5' primer comprises the unique NotI site at position 324, and the 3' primer carries a 5' end BglII site. In the second reaction, a MroI site is inserted at position 453 using oligonucleotides
SEQ ID NO 3: (hybridizing sequence: 448-480) and
SEQ ID NO 4: (hybridizing sequence: 905-881).

To allow subsequent cloning steps, a BglII site is added at the 5' end of the 5' primer, and the 3' primer is selected because it contains the unique BstEII site at position 888. The 128 bp and 469 bp PCR products digested with BglII are ligated together, then digested with NotI and BstEII and finally inserted into pSC-11 treated with the same two enzymes. To confirm the correct nature of the recombinant DNA, PCR-derived inserts are sequenced by the method of Sanger et al. (1977).

Several of the genes involved in invasion of cultured cells by *Shigella flexneri*, an enteropathogen that causes dysentery in humans, have been identified and designated as ipa for "invasion plasmid antigen" (Maurelli et al., 1985; Baudry et al., 1987). Two proteins encoded by ipa genes, IpaB and IpaC, are exposed on the bacterial surface, and might explain why antibodies to these proteins are seen in infected animals or recovering patients (Phalipon et al., 1992). For insertion into SC, we selected the immunodominant linear B cell epitope consisting of residues Lys-Asp-Arg-Thr-Leu-Ile-Glu-Gln-Lys (KDRTLIEQK) of IpaB (Barzu et al., 1993). The nine amino acid sequence is chosen in order to change the length of the original loop by only a single amino acid.

Oligonucleotides coding for the Shigella invasin B (IpaB) epitope (KDRTLIEQK) are synthesized with a 5' AccI half site on the coding strand (SEQ ID NO 5) and a 3' MroI half site on the non coding strand (SEQ ID NO 6).

Following annealing, the hybrid is introduced into the newly created AccI and MroI sites of plasmid pSC-11(A/M) (Fig. 4), The resulting construct is referred to as pSC(IpaB) (Fig. 5).

### Example 2: Construction of expression and insertion vectors pHGS1-SC6xHis and pHGS1-SC(IpaB)6xHis.

Epitope insertion is performed by inserting the fragment NotI (324)/SalI (1834) recovered from pSC(IpaB) into pLKneo-PIR (Hirt et al., 1992) digested with the same two enzymes. The resulting construct is called pLKneo-PIR(IpaB) (Fig. 5).

Rabbit SC cDNA is engineered to contain the coding sequences for amino acids 1 to 571 fused to a C-terminus histidine hexamer tag. The DNA fragment is cloned into VV insertion plasmid pHGS1 (Bertholet et al., 1985), which directs homologous recombination into the TK locus of the viral genome and contains regulatory sequences ensuring high expression levels in eukaryotic cells.

The insertion plasmids to generate rVVs are constructed as follows: Plasmid pSC-11 is digested with NcoI at position 122 and SalI at position 1834 (putative C-terminus; Mostov et al., 1984). The NcoI and SalI sites are filled in using Mungbean exonuclease, and the fragment is ligated into the blunt-ended EcoRI site of pHGS1 (Bertholet et al., 1985) containing the strong p11K late promoter inserted in the body of the viral TK gene. This results in the in frame fusion of the 11K ATG to the second codon of the SC coding region. Addition of a C-terminal six histidine tag is carried out by annealing of oligonucleotides SEQ ID NO 7 and SEQ ID NO 8 followed by insertion into the KpnI site located 3 bp upstream of the SalI site of pHGS1-SC. The sequence encodes 6 histidine residues, a translational termination codon, as well as XhoI and BglII sites to determine the orientation. The resulting plasmid is designated pHGS1-SC6xHis (Fig. 5).

Plasmid pHGS1-SC(IpaB)6xHis (Fig. 5) encoding antigenized SC is obtained by cleaving plasmid pHGS1-SC6xHis with NotI and BstEII and replacing the fragment by the corresponding mutated fragment coming from plasmid pSC(IpaB) (Fig. 5).

### Example 3: Generation of vaccinia virus recombinant vectors vvHGS1-SC6xHis and vvHGS1-SC(IpaB)6xHis.

Vaccinia virus recombinant vectors vvHGS1-SC6xHis and vvHGS1-SC(IpaB)6xHis are generated by homologous recombination into the thymidine kinase (TK) gene of constructs pHGS1-SC6xHis and pHGS1-SC(IpaB)6xHis as described (Rindisbacher et al., 1995). Integration of the SC gene construct in the viral genome is checked by PCR after each round of plaque purification using TK upstream primer SEQ ID NO 9 and TK downstream primer SEQ ID NO No 10.

### Example 4: Large scale production and purification of recombinant rabbit SC in CV-1 cells.

Stationary phase CV-1 cells (ATCC CCL 70) at a density of 2 x 10⁷ cells/175 cm² T-flask are washed with PBS, and infected with vaccinia virus recombinant vectors vvHGS1-SC6xHis and vvHGS1-SC(IpaB)6xHis expressing SC6xHis or SC(IpaB)6xHis, respectively, at a multiplicity of infection (M.O.I.) of 2. Infected cells are cultured in Dulbecco's modified Eagle's medium in the absence of serum and antibiotics for 24 hours. The culture supernatant is recovered by centrifugation at 120 x g for 15 min. The production of recombinant wild type and chimeric SC is determined by immunodetection (Rindisbacher et al., 1995).

Purification of secreted recombinant SC proteins is performed by Ni²⁺-chelate affinity chromatography according to the procedure given in Rindisbacher et al. (1995). The cell culture medium containing SC6xHis or SC(IpaB)6xHis is brought to pH 7.8, and subsequently loaded onto a column of Ni²⁺-nitrilotriacetic acid agarose (Qiagen) equilibrated in 20 mM phosphate sodium, 500 mM NaCl, pH 7.8. All purification steps are performed at 4^{o}C with prechilled buffers. One milliliter settled beads is used per 5 milligrams protein. Washing steps with 5 column volumes is carried out using equilibration buffer at pH 7.8 containing 8 mM imidazole. Elution is performed with equilibration buffer at pH 7.8 containing 40 mM and 80 mM imidazole. Fractions containing the purified protein are identified by SDS-PAGE with silver staining and immunodetection. Buffer exchange against PBS is carried out using Centriprep-50 units (Amicon), and the material is kept at 4^{o}C until further required.

For silver staining of recombinant SC, a selection of Ni²⁺-chelate column fractions are separated by SDS-PAGE, and then treated according to the procedure of Morrissey (1981).

For Western blot analysis of recombinat SC, a selection of Ni²⁺-chelate column fractions are separated by SDS-PAGE prior to transfer onto blotting membranes, and detected with the goat antiserum pAb 982 at a dilution of 1:500. Binding of goat antiserum pAb 982 is then detected using HRP-conjugated anti-goat IgG antibodies at a dilution of 1:3,000, and the chemio-luminescence detection reagent (Amersham).

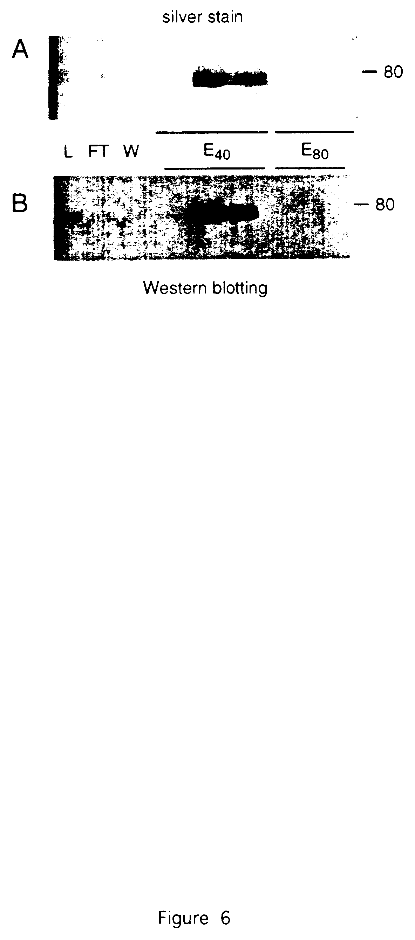

The results are shown in Figure 6 A and B.

### Example 5: Assay for binding rSC and IgAs.

The interaction between recombinant SC and IgA is determined by dot blot reassociation assay (DORA) as described in Rindisbacher et al. (1995). Murine IgA_{d} and IgAₘ are obtained from hybridoma ZAC3 (Lüllau et al., 1996).

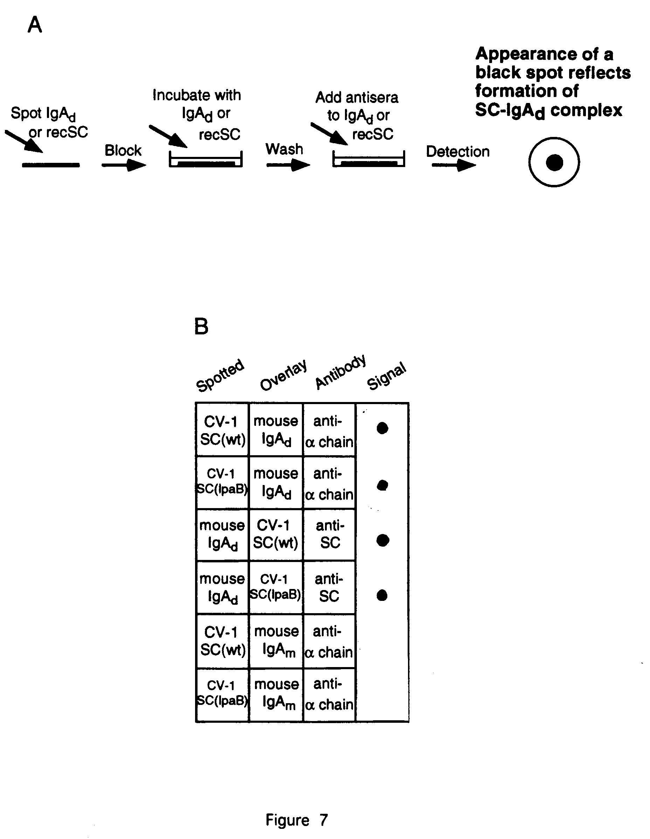

The results are shown in Figure 7A and B.

### Example 6: Reconstitution of IgA_{d}-SC(IpaB).

350 micrograms of SC(IpaB) or SC(wt) are associated in vitro with 1.75 milligrams of IgA_{d} purified from ZAC3 hybridoma (Lüllau et al., 1996) at room temperature in PBS. Reconstituted IgA_{d}-SC complexes are purified by FPLC (fast protein liquid chromatography system from Pharmacia) onto a 140 cm x 1.6 cm Superdex 200 (Pharmacia) column (Lüllau et al., 1996) equilibrated and eluted with PBS. Fractions containing the reconstituted sIgA are pooled and concentrated by filtration using Centriprep-50 units (Amicon) equilibrated in PBS. IgA_{d}-SC antibodies are sterilized by passage through a 0.2 micrometer pore size Acrodisc filter (Gelman Sciences) and stored at 4^{o}C until use.

### Example 7: Mucosal immunization with reconstituted IgA_{d}-SC(IpaB).

Immunization of mice with a mixture of 100 micrograms of in vitro reconstituted IgA_{d}-SC(IpaB) antibodies in PBS together with 10 micrograms of cholera toxin (Calbiochem) as an adjuvant in a final volume of 200 microliters of PBS demonstrates that chimerized reconstituted sIgA can serve as a mucosal delivery system. Germ-free BALB/c mice are administered the antibodies by intragastric intubation via PE10 polyethylene tubing connected to an 1 milliliter syringe. Two groups of three mice are challenged four times at day 1, 10, 44 and 51, and blood and saliva are collected 10 days after the last immunization. Biological fluids are kept frozen until use. Controls consist of immunization with 100 micrograms of IgA_{d}-SC(wt)/cholera toxin, and PBS buffer. Immune and control sera are collected and tested by enzyme linked immunosorbent assay (ELISA). *Shigella* lysates containing or lacking the IpaB antigen are applied to 96-well plates (Dynatech) to serve as the capture reagent for the antibodies in the sera to be tested. Wells are blocked with PBS-0.05% Tween-20 (Bio-Rad) for 30 minutes, and dilutions of the IpaB-specific antisera and control sera are added. Following incubation for 2 hours, the wells are washed with PBS-0.05% Tween-20, and binding of antibodies is measured by detection with a secondary antibody coupled to HRP. Read-out values correspond to the absorbance at 492 nanometers of the orthophenyl diamine substrate converted by HRP in each individual wells. High titers of antibodies to SC are obtained in sera from mice challenged with either IgA_{d}-SC(IpaB) or IgA_{d}-SC(wt) (Fig. 8A, left panel), thereby confirming the immunogenicity of SC when delivered into gastric mucosa. A strong specific response against the IpaB-containing lysate can be detected by incubation of the sera of the mice immunized with IgA_{d}-SC(IpaB) (Fig. 8A, middle panel). This indicates that serum antibodies in mice immunized with IgA_{d}-SC(IpaB) can recognize the IpaB epitope in its native environment. Background levels of similar low intensity are obtained with the sera of animals immunized with IgA_{d}-SC(wt) or PBS alone (Fig. 8A). No signal is obtained when a lysate lacking IpaB is coated (Fig. 8A, right panel). Sera from mice immunized with IgA_{d}-SC(IpaB) also bind to the free IpaB peptide, but not to the unrelated FLAG (IBI-Kodak) peptide (Fig. 8B). Saliva samples of two mice challenged with IgA_{d}-SC(IpaB) exhibits a reproducible, albeit weak binding to *Shigella* lysates containing IpaB (Fig. 8C). Antibodies against SC are detected in all saliva samples of animals immunized with IgA_{d}-SC(IpaB) or IgA_{d}-SC(wt) (Fig. 8C). Data in Fig. 8A-C thus demonstrate that gastric mucosa delivery of antigenized sIgA can serve as a vaccine system capable to elicit both a systemic and a mucosal antibody response.

The results are shown in Fig. 8 A-C.

### References

Banting, G., Brake, B., Braghetta, P., Luzio, J.P., and Stanley, K.K. (1989) FEBS Lett. **254**, 177-183
Barzu, S., Nato, F., Rouyre, S., Mazie, J.C., Sansonetti, P.J., and Phalipon, A. (1993) Infect. Immun. **61**, 3825-3831
Baudry B., Maurelli A. T., Clerc P., Sadoff J. C., and Sansonetti, P. J. (1987) J. Gen. Microbiol. **133**, 3403-3413
Bertholet, C., Drillien, R., and Wittek, R. (1985) Proc. Natl. Acad. Sci. U.S.A. **82**, 2096-2100
Bixler, G.S., Eby, R., Dermody, K., Woods, R.M., Seid, R.C., and Pillai, S., (1989) Immunobiol. Proteins Peptides **5**, 175-180
Burrows, S.R., Sculley, T.B., Misko, I.S., Schmidt, C., and Moss, D.J. (1990a) J. Exp. Med. **171**, 345-349
Burrows, S.R., Misko, I.S., Sculley, T.B., Schmidt, C., and Moss, D.J. (1990b) J. Virol. **64**, 3974-3976
Dion, A., Knittel, J., and Morneweck, S. (1990) Virology **179**, 474-477
deCampos-Lima, P.O., Levitsky, V., Brooks, J., Lee, S.P., Hu, L.F., Rickinson, A.B., and Masucci, M.G. (1994) J. Exp. Med. **179**, 1297-1305
Guillet, J.-G., Lai, M.-Z., Briner, T.J., Smith, J.A., and Gefter, M.L. (1986) Nature **324**, 260-262
Hanly, C.W., Cook, L., Kingzette, M., Cox, W., Frutiger, S., Hughes, G.J., and Jaton, J.-C. (1987) J. Immunol. **139**, 1597-1601
Hirt, R., Hughes, Poulain-Godefroy, O., Billotte, J., Kraehenbuhl, J.-P., and Fasel, N. (1992) Gene **111**, 199-206
Hoess, R.H. (1993) Curr. Opin. Structural Biology **3**, 572-579
Javaherian, K., Langlois, A.J., McDanal, C., Ross, K.L., Eckler, L.I., Jellis, C.L., Profy, A.T., Rusche, J.R., Bolognesi, D.P., Putney, S.D., and Matthews, T.J. (1989) Proc. Natl. Acad. Sci. U.S.A. **86**, 6768-6772
Kuhn, L.C., Kocher, H.P., Hanly, W.C., Cook, L., Jaton, J.-C., and Kraehenbuhl, J.-P. (1983) J. Biol. Chem. **258**, 6653-6659
Krajci, P., Grzeschik, K. H., Geurts van Kessel, A. H. M., Olaisen, B., and Brandtzaeg, P. (1991) Hum. Genet. **87**, 642-648
Labigne, A.F., Cussac, V., and Courcoux, P. (1991) J. Bacteriol. **173**, 1920-1931
Lüllau, E., Heyse, S., Vogel, H., Marison, I., von Stockar, U., Kraehenbuhl, J.-P., and Corthésy, B. (1996) J. Biol. Chem. **271**, 16300-16309
Maurelli, A.T., Baudry, B., d'Hauteville, H., Hale, T.L., and Sansonetti, P.J. (1985) Infect. Immun. **49**, 164-171
Morrissey, J.H. (1981) Anal. Biochem. 117, 307-310
Mostov, K. E., Friedlander, M., and Blobel, G. (1984) Nature **308**, 37-43
Neutra, M. R., Michetti, P., and Kraehenbuhl, J.-P. (1994) In Physiology of the Gastrointestinal Tract (Johnson, L. R., ed), p. 685, Raven Press, New York Panina-Bordignon, P., Tan, A., Termijtelen, A., Demotz, S., Corradin, G., and Lanzavecchia, A. (1989) Eur. J. Immunol. **19**, 2237-2242
Phalipon, A., Arondel, J., Nato, F., Rouyre, S., Mazie, J. C., and Sansonetti, P. J. (1992) Infect. Immun. **60**, 1919-1926
Rindisbacher, L., Cottet, S., Wittek, R., Kraehenbuhl, J.-P., and Corthésy, B. (1995) J. Biol. Chem. **270**, 14220-14228
Roy, M. J., and Varvayanis, M. (1987) Cell. Tissue Res. **248**, 645-651
Sanger, F., Nicklen, S., and Coulson, A.R. (1977) Proc. Natl. Acad. Sci. U.S.A. **74**, 5463-5467
Schaerer, E., Verrey, F., Racine, L., Talichet, C., Rheinhardt, M., and Kraehenbuhl, J.-P. (1990) J. Cell. Biol. **110**, 987-998
Schmidt, C., Burrows, S.R., Sculley, T.B., Moss, D.J., and Misko, I.S. (1991) Proc. Natl. Acad. Sci. U.S.A. **88**, 9478-9482
Solari, R., Kuhn, L.C., and Kraehenbuhl, J.-P. (1985) J. Biol. Chem. **260**, 1141-1145
Suerbaum, S., Thiberge, J.M., Kansau, I., Ferrero, R.L., and Labigne, A.F. (1994) Mol. Microbiol. **14**, 959-974
Taylor, P.M., Davey, J., Howland, K., Rothbard, J.B., and Askonas, B.A. (1987) Immunogenetics **26**, 267-272
Weltzin, R., Lucia-Jandris, P., Fields, B.N., Kraehenbuhl, J.-P., and Neutra, M.R. (1989) J. Cell. Biol. **108**, 1673-1685
Zaghouani, H., Krystal, M., Kuzu, H., Moran, T., Shah, H., Kuzu, Y., Schulman, J., and Bona, C. (1992) J. Immunol. **148**, 3604-3609

## Claims

**1.** A secretory IgA antibody comprising a polymeric IgA antibody bound to a secretory component which comprises a (poly)peptide insert corresponding to a protective epitope of an antigen..

**2.** A secretory antibody according to claim 1, wherein the protective epitope is inserted in a position where it does not inadequately interfere with the natural folding of the SC, where it does not inhibit the binding to the polymeric, especially dimeric IgA in vitro, and where it still retains its antigenic and immunogenic activity.

**3.** A secretory antibody according to claim 1, wherein the protective epitope of an antigen is inserted in a surface exposed side of the loop connecting the E and F β strand of domain I of rabbit SC.

**4. A secretory antibody according to claim 1, wherein the protective epitope of an antigen is fused to the carboxy or amino terminus of SC.**

**5. A secretory antibody according to claim 1, wherein a SC domain is replaced by a (poly)peptide carrying a protective epitope of an antigen.**

**6.** A chimeric secretory component which comprises as a (poly)peptide insert a protective epitope of an antigen.

**7.** A chimeric secretory component according to claim 6 wherein the (poly)peptide insert is a protective epitope of a pathogenic microorganism.

**8.** A chimeric secretory component according to claim 6 wherein the (poly)peptide insert is an amino acid sequence from a bacterial, viral, parasitic or mammalian antigen which is recognized by an immune effector, i. e. antibody, antigen presenting cell or lymphocyte.

**10.** A DNA construct comprising a DNA sequence coding for a secretory component and a DNA insert coding for the protective epitope of an antigen.

**11.** A DNA construct according to claim 10 comprising a DNA sequence coding for a secretory component and a DNA insert coding for the protective epitope of an antigen which is inserted at a position where the finally expressed foreign chimeric epitope in the secretory component does not effect the folding of the novel SC, the assembly with the polymeric IgA and the function of the novel sIgA as a vaccine delivery system.

**12.** A method for producing a secretory IgA antibody according to claim 1, wherein the secretory component according to claim 6 is combined with a polymeric IgA antibody.

**13. A method for purifying a secretory IgA antibody according to claim 1, and produced according to claim 12.**

**14.** A mucosal vaccine comprising a effective amount of a secretory IgA antibody according to claim 1.

**15.** A method for the active immunization of a mammal which comprises administering to said mammal an effective protecting amount of a vaccine according to claim 14.

**16. A method for the active immunization of a mammal according to claim 15, wherein the vaccine is administered via the nasal route, eg. in form of an aerosol or nasal drops; the oral route, e. g. in form of tablets or capsules, the vaginal or rectal route, e. g. in form of a suppository or an ointment.**


SEQUENCE LISTING

(1) GENERAL INFORMATION:
(i) APPLICANT:
(A) NAME: SWISS INSTITUTE FOR EXPERIMENTAL CANCER RESEARCH
(B) STREET: Ch. des Boveresses 155
(C) CITY: Epalinges s/Lausanne
(E) COUNTRY: Switzerland
(F) POSTAL CODE (ZIP): CH 1066
(G) TELEPHONE: xx41-21-6925858
(H) TELEFAX: xx41-21-6526933
(ii) TITLE OF INVENTION: Secretory Immunoglobulin A as a Mucosal Vaccine Delivery System
(iii) NUMBER OF SEQUENCES: 11
(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:
(i) SEQUENCE CHARACTERISTICS:
(A) LENGTH: 48 base pairs
(B) TYPE: nucleic acid
(C) STRANDEDNESS: single
(D) TOPOLOGY: linear
(ii) MOLECULE TYPE: other nucleic acid

(A) DESCRIPTION: /desc = "Oligonucleotide"
(iii) HYPOTHETICAL: NO
(iv) ANTI-SENSE: YES
(v) FRAGMENT TYPE: internal
(vi) ORIGINAL SOURCE:
(A) ORGANISM: Oryctolagus cuniculus
(vii) IMMEDIATE SOURCE:
(B) CLONE: pSC-11(A/M)
(viii) POSITION IN GENOME:
(B) MAP POSITION: 429-394
(C) UNITS: bp
(ix) FEATURE:
(A) NAME/KEY: primer_bind
(B) LOCATION:complement (13..48)
(xi) SEQUENCE DESCRIPTION: SEQ ID NO: 1:
GGGAAGATCT TCTAAGTATA CCACAAACTC CCCTTTTTCA GGGAAGTC 48

(2) INFORMATION FOR SEQ ID NO: 2:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: other nucleic acid
(A) DESCRIPTION: /desc = "Oligonucleotide"
(iii) HYPOTHETICAL: NO
(iv) ANTI-SENSE: NO
(v) FRAGMENT TYPE: internal
(vi) ORIGINAL SOURCE:
(A) ORGANISM: Oryctolagus cuniculus
(vii) IMMEDIATE SOURCE:
(B) CLONE: pSC-11(A/M)
(viii) POSITION IN GENOME:
(B) MAP POSITION: 314-337
(C) UNITS: bp
(ix) FEATURE:
(A) NAME/KEY: primer_bind
(B) LOCATION:complement (1..24)
(xi) SEQUENCE DESCRIPTION: SEQ ID NO: 2:
AAGAGGAGAG CGGCCGCTGC GTGA 24

(2) INFORMATION FOR SEQ ID NO: 3:
(i) SEQUENCE CHARACTERISTICS:
(A) LENGTH: 45 base pairs
(B) TYPE: nucleic acid
(C) STRANDEDNESS: single
(D) TOPOLOGY: linear
(ii) MOLECULE TYPE: other nucleic acid
(A) DESCRIPTION: /desc = "Oligonucleotide"
(iii) HYPOTHETICAL: NO
(iv) ANTI-SENSE: NO
(v) FRAGMENT TYPE: internal
(vi) ORIGINAL SOURCE:
(A) ORGANISM: Oryctolagus cuniculus
(vii) IMMEDIATE SOURCE:
(B) CLONE: pSC-11(A/M)
(viii) POSITION IN GENOME:
(B) MAP POSITION: 448-480
(C) UNITS: bp
(ix) FEATURE:
(A) NAME/KEY: primer_bind
(B) LOCATION:complement (13..45)
(xi) SEQUENCE DESCRIPTION: SEQ ID NO: 3:
GGGAAGATCT TCGACTCCGG AAGCTACAAG TGTGGCGTGG GAGTC 45

(2) INFORMATION FOR SEQ ID NO: 4:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: other nucleic acid
(A) DESCRIPTION: /desc = "Oligonucleotide"
(iii) HYPOTHETICAL: NO
(iv) ANTI-SENSE: YES
(v) FRAGMENT TYPE: internal
(vi) ORIGINAL SOURCE:
(A) ORGANISM: Oryctolagus cuniculus
(vii) IMMEDIATE SOURCE:
(B) CLONE: pSC-11(A/M)

(viii) POSITION IN GENOME:
(B) MAP POSITION: 905-881
(C) UNITS: bp
(ix) FEATURE:
(A) NAME/KEY: primer_bind
(B) LOCATION:complement (1..25)
(xi) SEQUENCE DESCRIPTION: SEQ ID NO: 4:
GCACATTCAA AGGTCACCGA GCCCC 25

(2) INFORMATION FOR SEQ ID NO: 5:
(i) SEQUENCE CHARACTERISTICS:
(A) LENGTH: 32 base pairs
(B) TYPE: nucleic acid
(C) STRANDEDNESS: single
(D) TOPOLOGY: linear
(ii) MOLECULE TYPE: other nucleic acid
(A) DESCRIPTION: /desc = "oligonucleotide"
(iii) HYPOTHETICAL: NO
(iv) ANTI-SENSE: NO
(v) FRAGMENT TYPE: internal
(vi) ORIGINAL SOURCE:
(A) ORGANISM: Shigella invasin B epitope KDRTLIEQK
(vii) IMMEDIATE SOURCE:
(B) CLONE: pSC(IpaB)
(viii) POSITION IN GENOME:
(A) CHROMOSOME/SEGMENT: Epitope KDRTLIEQK
(C) UNITS: bp
(ix) FEATURE:
(A) NAME/KEY: misc_feature
(B) LOCATION:5..31
(xi) SEQUENCE DESCRIPTION: SEQ ID NO: 5:
ATACAAAGAC AGGACATTGA TTGAGCAGAA AT 32

(2) INFORMATION FOR SEQ ID NO: 6:
(i) SEQUENCE CHARACTERISTICS:
(A) LENGTH: 34 base pairs
(B) TYPE: nucleic acid

(C) STRANDEDNESS: single
(D) TOPOLOGY: linear
(ii) MOLECULE TYPE: other nucleic acid
(A) DESCRIPTION: /desc = "oligonucleotide"
(iii) HYPOTHETICAL: NO
(iv) ANTI-SENSE: YES
(v) FRAGMENT TYPE: internal
(vi) ORIGINAL SOURCE:
(A) ORGANISM: Shigella invasin B epitope KDRTLIEQK (non coding strand)
(vii) IMMEDIATE SOURCE:
(B) CLONE: pSC(IpaB)
(viii) POSITION IN GENOME:
(A) CHROMOSOME/SEGMENT: Epitope KDRTLIEQK (non coding strand)
(C) UNITS: bp
(ix) FEATURE:
(A) NAME/KEY: misc_feature
(B) LOCATION:6..32
(xi) SEQUENCE DESCRIPTION: SEQ ID NO: 6:
   CCGGATTTCT GCTCAATAAC TGTCCTGTCT TTGT 34
(i) SEQUENCE CHARACTERISTICS:
(A) LENGTH: 38 base pairs
(B) TYPE: nucleic acid
(C) STRANDEDNESS: single
(D) TOPOLOGY: linear
(ii) MOLECULE TYPE: other nucleic acid
(A) DESCRIPTION: /desc = "oligonucleotide"
(iii) HYPOTHETICAL: NO
(iv) ANTI-SENSE: NO
(v) FRAGMENT TYPE: internal
(vi) ORIGINAL SOURCE:
(A) ORGANISM: six histidine tag
(vii) IMMEDIATE SOURCE:
(B) CLONE: pHGS1-SC6xHis
(viii) POSITION IN GENOME:
(C) UNITS: bp
(ix) FEATURE:
(A) NAME/KEY: misc_feature
(B) LOCATION:3..20

(xi) SEQUENCE DESCRIPTION: SEQ ID NO: 7:
CTCATCACCA TCACCATCAC TAAAGATCTC GAGTGTAC 38

(2) INFORMATION FOR SEQ ID NO: 8:
(i) SEQUENCE CHARACTERISTICS:
(A) LENGTH: 38 base pairs
(B) TYPE: nucleic acid
(C) STRANDEDNESS: single
(D) TOPOLOGY: linear
(ii) MOLECULE TYPE: other nucleic acid
(A) DESCRIPTION: /desc = "oligonucleotide"
(iii) HYPOTHETICAL: NO
(iv) ANTI-SENSE: YES
(v) FRAGMENT TYPE: internal
(vi) ORIGINAL SOURCE:
(A) ORGANISM: six histidine tag
(vii) IMMEDIATE SOURCE:
(B) CLONE: pHGS1-SC6xHis
(viii) POSITION IN GENOME:
(C) UNITS: bp
(ix) FEATURE:
(A) NAME/KEY: misc_feature
(B) LOCATION:15..32
(xi) SEQUENCE DESCRIPTION: SEQ ID NO: 8:
ACTCGAGATC TTTAGTGATG GTGATGGTGA TGAGGTAC 38

(2) INFORMATION FOR SEQ ID NO: 9:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: other nucleic acid
(A) DESCRIPTION: /desc = "oligonucleotide"
(iii) HYPOTHETICAL: NO
(iv) ANTI-SENSE: NO
(v) FRAGMENT TYPE: internal
(vi) ORIGINAL SOURCE:
(A) ORGANISM: pHGS 1 plasmid sequence
(viii) POSITION IN GENOME:

(C) UNITS: bp
(ix) FEATURE:
(A) NAME/KEY: primer_bind
(B) LOCATION:complement (1..23)
(xi) SEQUENCE DESCRIPTION: SEQ ID NO: 9:
GCTACGCTAG TCACAATCAC CAC 23

(2) INFORMATION FOR SEQ ID NO: 10:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: other nucleic acid
(A) DESCRIPTION: /desc = "oligonucleotide"
(iii) HYPOTHETICAL: NO
(iv) ANTI-SENSE: YES
(v) FRAGMENT TYPE: internal
(vi) ORIGINAL SOURCE:
(A) ORGANISM: vaccinia virus thymidine kinase gene
(ix) FEATURE:
(A) NAME/KEY: primer_bind
(B) LOCATION:complement (1..20)
(xi) SEQUENCE DESCRIPTION: SEQ ID NO: 10:
GTCCCATCGA GTGCGGCTAC 20

(2) INFORMATION FOR SEQ ID NO: 11:
(i) SEQUENCE CHARACTERISTICS:
(A) LENGTH: 9 amino acids
(B) TYPE: amino acid
(C) STRANDEDNESS: single
(D) TOPOLOGY: linear
(ii) MOLECULE TYPE: peptide
(iii) HYPOTHETICAL: NO
(iv) ANTI-SENSE: NO
(v) FRAGMENT TYPE: internal
(vi) ORIGINAL SOURCE:
(A) ORGANISM: Shigella invasin B epitope
(vii) IMMEDIATE SOURCE:
(B) CLONE: pHGS1-SC(IpaB)6xHis

(viii) POSITION IN GENOME:
(A) CHROMOSOME/SEGMENT: Shigella invasin B epitope
(ix) FEATURE:
(A) NAME/KEY: Peptide
(B) LOCATION:1..9
(xi) SEQUENCE DESCRIPTION: SEQ ID NO: 11:
Lys Asp Arg Thr Leu Ile Glu Gln Lys
1               5